(19) **Europäisches Patentamt**

**European Patent Office**

**Office européen des brevets**

(11) Publication number: **0 373 543 B1**

(12) **EUROPEAN PATENT SPECIFICATION**

(45) Date of publication of patent specification: **09.11.94**    (51) Int. Cl.5: **C12Q 1/18**

(21) Application number: **89122744.9**

(22) Date of filing: **09.12.89**

(54) **Diagnostic media and assay for detecting fungicide resistance.**

<table>
<tr><td>(30) Priority: <b>16.12.88 US 285658</b></td><td>(73) Proprietor: <b>BASF Corporation<br>9 Campus Drive<br>Parsippany, NJ 07054 (US)</b></td></tr>
<tr><td>(43) Date of publication of application:<br><b>20.06.90 Bulletin 90/25</b></td><td rowspan="2">(72) Inventor: <b>Bardinelli, Ted Robert<br>6 Greystone Court<br>Durham, NC 27713 (US)</b><br>Inventor: <b>Butterfield, Earle James<br>4507 Highgate<br>Durham, NC 27713 (US)</b><br>Inventor: <b>Jones, Timothy Lane<br>206 Bicket Blvd.<br>Raleigh, NC 27608 (US)</b></td></tr>
<tr><td>(45) Publication of the grant of the patent:<br><b>09.11.94 Bulletin 94/45</b></td></tr>
<tr><td>(84) Designated Contracting States:<br><b>AT BE CH DE ES FR GB GR IT LI NL SE</b></td></tr>
<tr><td>(56) References cited:<br><b>US-A- 4 489 161<br>US-A- 4 837 146<br>US-A- 4 882 273</b><br><br><b>BIOLOGICAL ABSTRACTS, vol. 71, no. 7, Biological Abstracts Inc., Philadelphia, PA (US); T. WATANABE et al., no. 48114&NUM;</b><br><br><b>PHYTOPATHOLOGY, vol. 79, no. 10, 1989, Washington, DC (US); T.R. BARDINELLI et al., pp. 1212-1213, no. 618&NUM;</b></td><td>(74) Representative: <b>Höller, Klaus, Dr. et al<br>BASF Aktiengesellschaft,<br>Patentabteilung ZDX - C 6<br>D-67056 Ludwigshafen (DE)</b></td></tr>
</table>

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a diagnostic media for detecting fungicide resistant strains of fungal plant pathogens and an assay, using this media, to detect such resistant stains of fungi.

The use of fungicides for controlling fungal plant pathogens in crops is an important and accepted way to maintain crops in a healthy condition throughout their growth cycle and improve yields. Changes in agricultural practices such as closer cropping sequences, breeding of new, higher-yielding cultivars, and use of heavier fertilizer dressings contribute to an increased incidence of fungal disease. The increasing demand for current commercial products and restrictions on the use of environmentally questionable compounds serves to accelerate the development of new fungicidally active synthetic organic compounds.

With the changes in agricultural practices and the increasing use of current commercial fungicides the problem of resistance build-up among fungal plant pathogens has developed and is a concern to both manufacturers of such pesticides and farmers. The expense and effort required to treat a crop with a fungicide should be undertaken with some confidence that the particular fungal plant pathogen is not resistant to the fungicidally active ingredient being applied.

Present methods of determining fungicide resistance in crops are by time consuming and labor intensive means of collecting infected tissue samples, then isolating each individual strain and then plating each individual strain on a nutrient medium containing the fungicide. The plates are then incubated for a period of time and then microscopically examined for growth of the spores. Alternatively, macroscopic evaluations are possible after incubation for about 5 days. Large numbers of isolates must be tested to insure accuracy of the observations of growth.

New methods and means for rapidly and accurately detecting the sensitivity and resistance of fungal plant pathogens to fungicidally active synthetic organic compounds are highly desirable. In Phytopathology, 74, no. 12 (1984) 1423-1425, a device for sampling spores to monitor fungicide resistance in the field is disclosed. The sampling devise is a lipbalm tube containing a core of solidified water agar or water agar including a fungicide. The agar core is extruded above the top of the vial by turning the dispensing knob at the vial base. The exposed moist agar surface is gently pressed against a plant lesion in the field and the vial is capped. After completion of field sampling the vials are returned to a laboratory for further processing. The extended portion of each agar core is sliced off flush with the top of the vile and placed in a petri dish. After incubation, the spores on each disc are observed with an incident light microscope for germination.

It is known to stain agar plates inoculated with conidia of Trichoderma viride with lacto-fuchsin and to count germinated and nongerminated conidia (US-4,489,161).

Bacteriological methods useful for isolation, identification and determination of characteristic properties of bacteria are known. Such bacteriological methods, and media associated with those methods, are not necessarily useful for fungi at least in part due to one or more of different life cycles, colony characteristics, physiology and nutritional requirements.

The diagnostic media of the present invention permits rapid determination of fungicide resistance in one simple plating. This, of course, saves a significant amount of time, labor and money. Greater accuracy is afforded as more strains can be tested in a given time than by the known methods. The diagnostic media can be used with equal effectiveness in the laboratory or the field and without the need for any special equipment. Particularly advantageous, is that fungicide resistance is determined without the need for microscopic examination.

It is therefore, an objective of the present invention to provide a diagnostic media and assay using said media, for field and laboratory evaluations, permitting the rapid and inexpensive determination of fungicide resistant fungal plant pathogens, particularly in fruit and vegetable crops. It is a further objective that fungicide resistance can be determined without the need for microscopic examination.

In accordance with the present invention, there is provided a diagnostic media for detecting fungicide resistant fungal plant pathogens comprising: an acid-base indicator dye; a fungicide selected from the group consisting of two-ring dicarboximides, benzimidazole carbamates, and biscarbamates; a carbohydrate; and water.

Also in accordance with the present invention there is provided a method for detecting fungicide resistance in fungal plant pathogens comprising innoculating the diagnostic media of the present invention with fungal spores; incubating said innoculated media; and macroscopically observing a color change of the media to detect the presence of fungicide resistant fungal plant pathogens.

The diagnostic media of the present invention permits rapid determination of synthetic organic fungicide resistance in one simple plating. Once plated on the media, only resistant fungal plant pathogens will germinate. Upon germination of the spores, the pH of the medium is changed causing the acid base

2

indicator dye in the medium to change color. This macroscopically observable color change can be seen in as early as about 18 hours or observations may be made as late as about 96 hours, but preferably observations are made after about 36 hours of incubation up to about 60 hours of incubation and most preferably after about 48 hours. The particular incubation temperature and humidity will vary depending upon the growth requirements of the particular fungal plant pathogen Species under investigation. Such growth requirements are known by or can be readily ascertained by one skilled in the art.

The diagnostic media of the present invention can be used to detect fungicide resistance in fungal plant pathogens which infest horticultural fruit and/or vegetable crops. More particularly, the diagnostic media and assay of the present invention can be used to ascertain fungicide resistance in ascomycete fungal plant pathogens having imperfect reproductive forms; that is asexual conidia. Representative examples of such fungal plant pathogens include Sclerotinia spp. such as Botrytis cinerea, Monilia laxa, Monilia fructigena, and Monilia fructicola (Monilia spp. are referred to at times herein as Monilinia spp.); Venturia spp. such as Fusicladium inaequalis, Fusicladium pirina and Fusicladium cerasi; Eurotium spp. such as Aspergillus niger, Aspergillus flavus, Penicillium expansum, Penicillium digitatum, and Penicillium italicum.

The acid-base indicator dye constituent of the diagnostic media of the present invention is generally any of the known organic substances which indicates by a change in its color the presence or absence of acids and bases. In the context of the present invention, those acid-base indicator dyes which will change color to indicate the presence of an acid increase in the medium are preferred. Representative examples of such useful acid-base indicator dyes include bromcresol purple, cresol red, bromcresol green, bromphenol blue, bromthymol blue, bromothymol blue, Congo red, methyl red, and the like. The particular choice of acid-base indicator dye depends on the range of pH indication and buffering capacity of the dye. The concentration of dye necessary to achieve an acceptable color change should not be inhibitory to the growth of the fungal plant pathogen being evaluated or adversely impact on the stability of the particular fungicide. Generally from about 0.1 g/liter to about 2 g/liter of acid base indicator dye affords acceptable color change at a reasonable cost, and preferably from about 0.2 to about 1.0 g/liter is used. Preferred acid-base indicator dyes are bromcresol purple and bromthymol blue and particularly preferred is bromcresol purple.

The pH of the diagnostic media of the present invention should be from about 5 to about 6 and may be adjusted, preferably using 0.01 N sodium hydroxide although any suitable inorganic alkalizing agent may be used. In a particular application it is possible to go above or below this pH range depending upon the particular indicator dye chosen and the growth requirements of a particular organism. Preferably the diagnostic media of the present invention will have a pH from about 5.1 to about 5.5.

The fungicidally active ingredient of the diagnostic media of the present invention will generally be from the group consisting of the 2-ring dicarboximide fungicides, for example fused rings fungicides such as captan, captafol and folpet and bonded rings fungicides such as chlorzolinate, procymidone, iprodione, and vinclozolin; benzimidazole carbamates such as benomyl and carbendazim; and biscarbamates such as thiophanate and thiophanate-methyl. Preferably the fungicide constituent of the diagnostic media of the present invention will be a 2-ring dicarboximide group or a benzimidazole carbamate and most preferably will be a bonded rings dicarboximide group containing fungicide or benomyl. Generally, the fungicide will be present at from about 2.5 ppm to about 150 ppm, preferably from about 10 ppm to about 100 ppm.

The carbohydrate constituent of the diagnostic media of the present invention can be a monosaccharide, oligosaccharide (2 to 10 monosaccharide units joined in glycosidic linkage), or a polysaccharide. Preferably the carbohydrate is a monosaccharide and the preferred monosaccharide is a hexose and particularly preferred is dextrose (glucose). Where an oligosaccharide is present, the monosaccharide units which are joined to form the oligosaccharide will consist of 6 carbon atom (hexose) monosaccharides. When the carbohydrate is a polysaccharide preferably it will be starch. The amount of carbohydrate obtained in the diagnostic media of the present invention will vary and must be adjusted to accommodate the nutritional requirements of a particular fungal plant pathogen but not be so high as to inhibit spore germination or fungal growth. It will be appreciated that combinations of the carbohydrates are also useful in the diagnostic media of the present invention. It should be appreciated that the concentration of the particular carbohydrate can enhance the selectivity of the medium.

Although any of the above-described saccharides, alone or in combination, may be used as the carbohydrate constituent, as the chain length increases from oligosaccharide to polysaccharide, the selectivity of the media diminishes. More false positive color changes are noted when potato dextrose agar (PDA) media is used as distinguished from water-dextrose agar. It is believed that PDA media sustains fungal growth for contaminant fungi to a greater extent than water-dextrose agar. These contaminant fungi cause the false positive color change. Typically, greater selectivity for the media is preferred, thereby affording greater reliability of the assay.

3

The water constituent of the diagnostic media of the present invention will preferably be deionized to help avoid chemical contamination.

Although not essential, it is highly preferred that a single broad spectrum antibiotic be included in the diagnostic media of the present invention. The presence of a broad spectrum antibiotic, of course, serves to help eliminate any bacterial contamination which may occur, particularly during inoculation. Generally from about 20 ppm to about 80 ppm and preferably from about 35 ppm to about 65 ppm of the broad spectrum antibiotic will be incorporated. Although it is believed any broad spectrum antibiotic may be used, preferred antibiotics are streptomycin sulfate and tetracyclin and particularly preferred is streptomycin sulfate. When present in the diagnostic media of the present invention, it is preferred that a single antibiotic is used, rather than mixtures of antibiotics.

The selectivity of the diagnostic medium of the present invention can be improved by the addition of a selective fungal growth inhibitor. The presence of a selective fungal growth inhibitor, of course, serves to inhibit growth of undesirable fungal contaminants which may occur, particularly as a consequence of inoculation. The selection and use of a particular inhibitor or combinations of inhibitors would be readily apparent to and known by those skilled in the art. Representative examples of such inhibitors include metalaxyl, cycloheximide, nystatin, penta-chloronitrobenzene (PCNB), rose bengal, gallic acid, oxgall, fenaminosulf, and ergosterd biosynthesis inhibitors such as triazole derivatives and imidazole derivatives. Generally, a single fungal growth inhibitor will be used rather than combinations of such agents although for a particular application combinations of such agents may be preferred. From about 10 ppm to about 500 ppm of the fungal growth inhibitor will be incorporated into the medium where such an agent is used. Some minimal experimentation may be necessary or desirable to optimize the amount of a particular fungal growth inhibitor used in a given diagnostic medium composition according to the present invention. Such experimentation would be routine and readily apparent to one skilled in the art.

Although it is believed the diagnostic media of the present invention may be advantageously utilized as a broth, it is highly preferred that agar be included in the composition. The agar, of course, permits the media to be plated and can be handled much more conveniently. The amount of agar necessary to gel a particular formulation is well known to those skilled in the art or can be readily ascertained.

Although a nonselective nutrient medium such as potato dextrose agar (PDA) may be used, the number of false positive color changes obtained when PDA is included in the diagnostic media significantly reduces the reliability of the assay.

Generally the diagnostic media of the present invention should be prepared shortly before use. The fungicide component of the media may hydrolize or otherwise break down to a fungicidally inert compound over extended periods of time, dependent of course, on factors such as pH and temperature. If stored at room temperature, the media should be used within about a week and if stored at about 5°C the media should be used within about 6 weeks.

The diagnostic media of the present invention may be prepared by methods well known to those skilled in the art. To avoid any ambiguity in this regard, Preparation Examples 1 and 2 below describe preparation of the diagnostic media of the present invention.

Preparation Example 1

Deionized water is added to 0.4 g bromcresol purple (dibromo-o-cresolsulfonphthalein) dissolved in 100 ml of 0.01N NaOH to bring the total volume to 800 ml. The pH is adjusted to about 5.3 using 0.01N NaOH. To this solution 20 g of agar is added. The solution is autoclaved. After autoclaving the solution is cooled to 50°C and 40g of dextrose dissolved in 200 ml of deionized water which has been filter sterilized is added and the combined solutions are mixed by swirling. To this combined solution either a 40 mg solution of vinclozolin dissolved in 5 ml of ethanol or a solution of 10 mg of benomyl dissolved in 5 ml of ethanol is added, and a solution of 50 mg of streptomycin sulfate in 5 ml of ethanol is also added and the combined solutions swirled to mix. The diagnostic media is then dispensed immediately into petri plates. The vinclozolin containing media is referred to at times herein as "VRA" and the benomyl containing media as "BRA". The above diagnostic media constitutes the best mode of the present invention.

Preparation Example 2

To 1 liter of deionized water, 39 grams of potato dextrose agar (PDA) powder commercially available from Difco Laboratories, Inc., Detroit, Michigan 48232 is added and the solution sterilized in an autoclave for 15 minutes. The solution is allowed to cool to about 50°C. To this solution either a 10mg solution of vinclozolin dissolved in 5 ml of ethanol or a solution of 10 mg of benomyl dissolved in 5 ml of ethanol is added, and a solution of 50 mg of streptomycin sulfate in 5 ml of ethanol is also added and the combined solutions swirled to mix. The media is then dispensed immediately into petri plates. The vinclozolin containing media is referred to at times herein as "PDAV" and the benomyl containing media as "PDAB".

It has been found by biological evaluation that the diagnostic media of the present invention effectly distinguishes resistant from sensitive strains of fungal plant pathogens. These fungal plant pathogens, particularly those previously described are destructive pests in horticultural fruit and vegetable crops. Biological evaluation procedures and results are described below.

Resistant and sensitive fungi were initially obtained from crops of cultivated fruit in the field. The percent germination reported below was calculated by microscopic evaluation. An average of four fields of view per plate was examined with each field of view having a total of about 100 to 300 spores. The total number of spores in each field of view was counted then either the number germinated or not germinated, whichever was easiest to count, was determined. These numbers were then combined appropriately and the percent germination calculated. Unless otherwise noted, resistant isolates are given a numerical designation and sensitive isolates a letter designation.

Example 1

Botrytis cinerea of known sensitivity and resistance to vinclozolin were cultured on potato dextrose agar prepared according to manufacturer's directions and Preparation Example 2 for 2 weeks and induced to sporulate under long wave ultraviolet light. Spores were transferred using a sterile cotton tipped swab to a solid medium containing 20 g of agar, 20 g dextrose and the amount of vinclozolin in parts per million per liter of media shown in Table 1 below. The innoculated media was incubated at room temperature for 18 hours and microscopically examined for germination; the results of this assay are set forth below in Table 1 where "BC" means Botrytis cinerea.

TABLE I

| Isolate No. | Percent Germination | | | |
|---|---|---|---|---|
| | 0 | 2.5 | 5.0 | 10.0 |
| BC-1 resistant | 100 | 100 | 100 | 100 |
| BC-2 res. | 100 | 100 | 100 | 100 |
| BC-3 res. | 100 | 100 | 90 | 80 |
| BC-4 res. | 100 | 100 | 100 | 100 |
| BC-5 res. | 100 | 100 | 100 | 90 |
| BC-6 res. | 100 | 100 | 100 | 100 |
| BC-A sensitive | 100 | 0 | 0 | 0 |
| BC-B sens. | 100 | 0 | 0 | 0 |
| BC-C sens. | 100 | 0 | 0 | 0 |
| BC-D sens. | 100 | 0 | 0 | 0 |

The data in Table I demonstrates that at 2.5 ppm of vinclozolin, spores from resistant strains of Botrytis cinerea germinated while spores from sensitive strains were unable to germinate.

Example 2

To a stock solution of 0.4 g bromcresol purple in 260 ml of 0.01N sodium hydroxide, either 40 g of potato dextrose agar (PDA) or 40 g dextrose and 20 g agar (WA-dextrose medium) were added and the total volume brought up to 1 liter using distilled water. After autoclaving the media, a solution of vinclozolin in 5 ml of ethanol was added to afford 5 or 10 ppm concentrations and dispensed into petri plates (10 ml/plate). Sporulating cultures of Botrytis cinerea and Rhizopus nigricans (also known as Rhizopus stolonifer) were grown for two weeks on potato dextrose agar. Spores were transferred to the diagnostic media using sterile cotton tipped swabs and incubated for 20 hours at room temperature. The media was macroscopically examined and the diameter of color change detectable in the media was then measured. The results are shown below in Table II.

TABLE II

| Isolate # | COLOR CHANGE DIAMETER (mm) | | | |
|---|---|---|---|---|
| | WA-dextrose media | | PDA media | |
| | 5.0ppm | 10.0ppm | 5.0ppm | 10.0 ppm |
| BC-A sensitive | 0 | 0 | 0 | 0 |
| BC-D sens. | 0 | 0 | 0 | 0 |
| BC-B sens. | 0 | 0 | 0 | 0 |
| BC-3 resistant | 10 | 10 | 10 | 0 |
| BC-2 res. | 10 | 5 | 10 | 0 |
| BC-1 res. | T[a] | 5 | 5 | 0 |
| RP[b]-1 | 0 | 0 | T | 0 |
| RP-2 | 0 | 0 | T | 0 |

[a]T = only slight color change.
[b]RP = Rhizopus nigricans)

As the data in Table II evidences, at 20 hours, the water-dextrose medium containing 10 ppm vinclozolin allowed excellent color change for only resistant isolates of Botrytis cinerea. The potato dextrose agar (PDA) allowed some color change for the Rhizopus nigricans, which is a common fungal contaminant at 5 ppm of vinclozolin. Incubation was allowed to continue for a total of 4 days incubation period. After 4 days, observations showed the water dextrose media allowed only the resistant isolates of Botrytis cinerea to produce a 25 mm diameter color reaction, while the PDA media containing 5 ppm of vinclozolin allowed 1 of the Rhizopus nigricans contaminated isolates to react. The PDA media containing 10 ppm of vinclozolin showed no measurable color change for any of the resistant isolates.

Example 3

Diagnostic media was prepared substantially as described above in Preparation Example 1 except that bromothymol blue rather than bromcresol purple and only 4 g of dextrose were used per liter and the pH adjusted to 7.5, 8.0 and 8.5. These media were then inoculated with spores of either Botrytis cinerea or Rhizopus nigricans isolates substantially as previously described. The inoculated media was then incubated for 18 hours at room temperature and the results recorded below in Table III.

TABLE III

DIAMETER OF COLOR CHANGE (mm)

| Isolate # | pH: | 7.5 | 8.0 | 8.5 |
|---|---|---|---|---|
| BC-A sensitive | | 0 | 0 | 0 |
| BC-C sens. | | T | 0 | 0 |
| BC-D sens. | | T | 0 | 0 |
| BC B sens. | | 0 | 0 | 0 |
| BC-6 resistant | | 6 | 3 | 6 |
| BC-5 res. | | 6 | 3 | 6 |
| BC-4 res. | | 12 | 12 | 12 |
| BC-3 res. | | 18 | 12 | 12 |
| BC-2 res. | | 12 | 6 | T |
| BC-1 res. | | 12 | 6 | 0 |
| RP-3 | | T | T | T |
| RP-4 | | T | T | T |

The above plates were also microscopically examined and showed greater than 90% spore germination for all of the resistant isolates and no germination of the sensitive isolate spores. Strong color reactions were only observed from the resistant <u>Botrytis cinerea</u> isolates. Only slight reactions, at most, occurred from the <u>Rhizopus nigricans</u> inoculations.

<u>Example 4</u>

Diagnostic media was prepared substantially as described above in Preparation Example 1 and the pH adjusted to 5.5 except that the vinclozolin concentrations in the media varied from about 10 ppm to 1000 ppm. The media was inoculated with 1 vinclozolin sensitive (BC-A) and 1 vinclozolin resistant isolate (BC-4) of <u>Botrytis cinerea</u> and incubated at room temperature for about 48 hours. The results are shown below in Table IV.

TABLE IV

| Vinclozolin Concentration | | |
|---|---|---|
| vinclozolin concentration (ppm) | Color Change Response | |
| | BC-A | BC-4 |
| 10 | - | + + + |
| 20 | - | + + + |
| 40 | - | + + + |
| 60 | - | + + + |
| 80 | - | + + + |
| 100 | - | + + + |
| 200 | + + + | + + + |
| 500 | - | T |
| 1000 | T | T |

As the data in the above table demonstrates concentrations of vinclozolin above 100 ppm tended to cause an acidic response from the sensitive BC-A spores without germination, leading to false positives. Spores from BC-4, the vinclozolin resistant isolate germinated in and grew causing good color responses at all concentrations between 10 and 200 ppm.

<u>Example 5</u>

Diagnostic media containing either vinclozolin (VRA) or benomyl (BRA) were prepared substantially as described above in Preparation Example 1. Isolates of <u>Botrytis cinerea</u> that had previously been tested for resistance or sensitivity to vinclozolin, benomyl, or both by microscopic examination were grown on potato dextrose agar and used to inoculate the diagnostic media. The innoculated plates were incubated for 48 hours at room temperature and then visually examined. The results are set forth below in Table V.

TABLE V

| Isolate # | Resistance | Color Change Rating | |
|---|---|---|---|
| | | BRA | VRA |
| BC-E | none | - | - |
| BC-15 | B and V | + + + + | + + + + |
| BC-16 | B and V | + + + + | + + + + |
| BC-7 | B | + + + + | - |
| BC-8 | B | + + + | - |
| BC-17 | B and V | + + + | + + + |
| BC-9 | B | + + + + | - |
| BC-10 | B | + + + | - |
| BC-11 | B | + + + | - |
| BC-18 | B and V | + + + + | + + + + |
| BC-F | none | - | T |
| BC-G | none | - | - |
| BC-12 | B | + + + | - |
| BC-19 | B and V | + + | + + + + |
| BC-13 | B | + + + | - |
| BC-14 | B | + + + | - |

Data from the above evaluations demonstrates an excellent correlation between color change and resistance.

Example 6

VRA and BRA diagnostic media were prepared substantially as described in Preparation Example 1 except that 10 ppm of vinclozolin or benomyl was used. Potato dextrose agar media was also prepared containing vinclozolin (designated as PDAV) or benomyl (designated as PDAB) at 10 ppm, as described in Preparation Example 2. Fungal contaminants from fresh strawberries and raspberries were isolated and grown on potato dextrose agar media. Spores from these fungal contaminant isolates were transferred to the test media using sterile cotton tipped swabs. The innoculated media was incubated for 4 days at room temperature and then visually observed. The results are set forth below in Table VI. These contaminants were identified only as to genera and not as to species. For these instances where more than one species of an identified generea were used, each species is distinguished by a numerical designation.

**TABLE VI**

| Strawberry Contaminants | Media | Observations |
|---|---|---|
| Cladosporium spp. | VRA: | small dark colonies 1.59mm(1/16 inch) diameter no color reaction |
| | BRA: | small dark colonies 1.59mm(1/16 inch) diameter no color reaction |

| | | | |
|---|---|---|---|
| | | PDAV: | 12.7mm(1/2 inch) diameter growth |
| | | PDAB: | 6.35mm(1/4 inch) diameter growth |
| _Colletotrichum spp._ | | VRA: | no growth or color reaction |
| | | BRA: | no growth or color reaction |
| | | PDAV: | 12.7mm(1/2 inch) diameter growth |
| | | PDAB: | no growth |
| _Rhizopus spp._ | 1 | VRA: | no growth or color reaction |
| | | BRA: | sparse growth and good color reaction |
| | | PDAV: | growth covered plate |
| | | PDAB: | growth covered plate |
| | 2 | VRA: | no growth or color reaction |
| | | BRA: | slight growth and alkaline color reaction |
| | | PDAV: | growth covered plate |
| | | PDAB: | growth covered plate |
| | 3 | VRA: | trace color reaction and no growth |
| | | BRA: | sparse growth and good color reaction |
| | | PDAV: | growth covered plate |
| | | PDAB: | growth covered plate |

**Raspberry
Contaminants**

| | | | |
|---|---|---|---|
| **Cladosporium spp.** | 1 | VRA: | small dark colonies 1.59mm(1/16 inch) diameter |
| | | BRA: | small dark colonies 1.59mm (1/16 inch) diameter |
| | | PDAV: | 12.7mm(1/2 inch) diameter growth |
| | | PDAB: | 25.4mm(1 inch) diameter growth |
| | 2 | VRA: | small dark colonies 1.59 mm(1/16 inch) diameter |
| | | BRA: | small dark colonies 1.59mm(1/16 inch) diameter |
| | | PDAV: | 12.7mm 1/2 inch diameter growth |
| | | PDAB: | 25.4mm 1 inch diameter growth |
| **Rhizopus spp.** | 1 | VRA: | no growth or color reaction |

9

| | | | |
|---|---|---|---|
| | **BRA:** | sparse growth and good color reaction | |
| | **PDAV;** | growth covered plate | |
| | **PDAB:** | growth covered plate | |
| **2** | **VRA:** | no growth or color reaction | |
| | **BRA:** | no growth or color reaction | |
| | **PDAV:** | growth covered plate | |
| | **PDAB:** | growth covered plate | |
| **3** | **VRA:** | no growth or color reaction | |
| | **BRA:** | sparse growth and good color reaction | |
| | **PDAV:** | growth covered plate | |
| | **PDAB:** | growth covered plate | |

As the above data demonstrates Rhizopus spp. failed to cause color reactions on vinclozolin media (VRA). On benomyl media (BRA) some Rhizopus spp. isolates may cause color reactions, but it is easy to visually identify this contaminant by its sparse growth and black globose sporophores. Cladosporium spp. was virtually eliminated on the vinclozolin (VRA) and benomyl (BRA) media while on the potato dextrose agar vinclozolin (PDAV) and potato dextrose agar benomyl (PDAB) media growth was moderate. Colletotrichum spp. growth was inhibited on both the vinclozolin (VRA) and benomyl (BRA) media but it grew well on the potato dextrose agar vinclozolin (PDAV) media although not on the potato dextrose agar benomyl (PDAB) media. Since benomyl is active against this fungus, such a result was expected. In contrast, the vinclozolin is not active against this fungus demonstrating the selectivity of the media (VRA) against Colletotrichum spp.. The potato dextrose agar amended with either vinclozolin or benomyl allows many contaminants to grow and this growth is often heavy, rendering it extremely difficult if not impossible to macroscopically observe slower growing Botrytis cinerea or Monilinia spp. fungal plant pathogens.

Example 7

Spores from brown rot Monilinia spp. infected peaches were sampled using sterile cotton swabs. These samples were brought to the laboratory and immediately plated on benomyl containing (BRA) diagnostic media prepared substantially according to Preparation Example 1 and also on potato dextrose agar containing benomyl (PDAB) media at 10 ppm concentrations. A total of 48 samples were plated at 4 samples per plate. The plates were incubated for 4 days at room temperature and then visually observed. The results are set forth below in Table VII. Completely negative results are not included in Table VII.

10

TABLE VII

| Sample # | BRA media color reaction | PDAB growth |
|---|---|---|
| 13 | - | CL,RH |
| 14 | - | CL,RH |
| 15 | - | CL,RH |
| 16 | - | CL,RH |
| 18 | + + + | MF |
| 27 | - | RH |
| 28 | + + + | RH |
| 32 | + + + | MF |
| 37 | - | Bact. |
| 38 | - | Bact. |
| 39 | - | RH |
| 43 | + + + | RH |
| 45 | - | RH |
| 46 | - | RH |
| 47 | - | RH |
| 48 | - | RH |

Notes: + + + = strong color reaction. CL = Cladosporium spp., RH = Rhizopus spp., MF = Monilinia spp., and Bact. = bacteria (all except MF are contaminants).

As the above data demonstrates, only 2 of the 48 samples collected were benomyl resistant (samples No. 18 and 32). There was excellent correlation between the media for detection of resistance. Although both media detected resistance, the potato dextrose agar benomyl (PDAB) medium was contaminated in 14 of the 48 platings. The benomyl media (BRA) only gave false positive color reactions for 2 of the Rhizopus contaminants. These contaminants were easily distinguished from the Monilinia growth by the Rhizopus sparse growth and black sporophores and the Monilinia spores can still be observed through the Rhizopus contaminant. As before, the contaminants were identified only as to genera and not as to species.

Example 8

Benomyl (BRA) media and potato dextrose agar media containing benomyl (PDAB) were prepared substantially as described in Preparation Examples 1 and 2. The concentration of benomyl in both media was 10 ppm. Strawberries infected with Botrytis cinerea were sampled using sterile cotton swabs to collect the spores and transfer them by innoculation to the surface of the media. There were 100 samples plated. These plates were incubated for 4 days at room temperature. The data for the resistant samples is set forth below in Table VIII.

TABLE VIII

| Isolate # | BRA color reaction | PDAB Botrytis growth (mm) |
|---|---|---|
| 1 | + + + | 55 |
| 2 | + | 10 |
| 8 | + + + + | 70 |
| 11 | + + + | 42 |
| 15 | - | 15 |
| 16 | + + + + | 72 |
| 26 | + + | 52 |
| 28 | + + + + | 54 |
| 57 | trace | 15 |
| 58 | + | 65 |
| 64 | + | 42 |
| 82 | + | 45 |
| 84 | + | 43 |
| 93 | + | 42 |
| 94 | + | unknown contaminant |

Benomyl resistance was detected in about 13% of the samples. Sample No. 15 did not cause a color reaction on benomyl (BRA) media and was able to make a little growth on the potato dextrose agar benomyl (PDAB) media. Sample number 94 , was an unknown fungal contaminant that was able to cause a false positive on the benomyl (BRA) media. Contamination on the potato dextrose agar and benomyl (PDAB) media was about 39%, of which 24% was due to Cladosporium spp.. The rest of the contamination on potato dextrose agar and benomyl (PDAB) media was due to various organisms including Colletotrichum spp., Rhizopus spp., Alternaria spp., bacteria and several unknown fungi. As previously noted, several of the unknown fungal contaminants were identified only as to generea and not as to species.

Example 9

Vinclozolin containing media (VRA) and benomyl containing media (BRA) were prepared substantially according to the procedures described in Preparation Example 1. Potato dextrose agar and vinclozolin (PDAV) media and potato dextrose agar and benomyl (PDAB) media containing 10 ppm of either vinclozolin or benomyl were also prepared substantially as described in Preparation Example 2. Botrytis cinerea isolates of known sensitivity or resistance to benomyl and vinclozolin were plated on these media using sterile cotton tipped swabs. These plates were incubated for 24 hours and then percent germination was determined; incubation was continued for an additional 24 hours after which time the vinclozolin (VRA) media and benomyl (BRA) media were rated for color reactions. The results are set forth below in Table IX.

TABLE IX

| Isolate # | %Germination | | | | Color Reaction | |
|---|---|---|---|---|---|---|
| | VRA | PDAV | BRA | PDAB | VRA | BRA |
| BC-H | 0 | 0 | 0 | 0 | - | - |
| BC-I | 0 | 0 | 0 | 0 | - | - |
| BC-20 | 0 | 0 | 8 | 12 | - | - |
| BC-21 | 0 | 0 | 90 | 90 | - | + + + |
| BC-22 | 0 | 0 | 90 | 90 | - | + + + + |
| BC-23 | 0 | 0 | 75 | 80 | - | + + + |
| BC-M | 0 | 0 | 0 | 0 | - | - |
| BC-25 | 90 | 90 | 90 | 90 | + + + + | + + + + |
| BC-26 | 0 | 0 | 30 | 20 | - | + + + |
| BC-27 | 90 | 85 | 90 | 90 | + + + + | + + |
| BC-28 | 90 | 90 | 85 | 90 | + + | + + + |
| BC-29 | 0 | 0 | 25 | 30 | - | + |
| BC-30 | 65 | 50 | 85 | 80 | + + | + + |
| BC-31 | 0 | 0 | 65 | 75 | - | + + + |
| BC-32 | 5 | 0 | 90 | 85 | T | + + + |
| BC-J | 0 | 0 | 0 | 0 | - | - |
| BC-K | 0 | 0 | 0 | 0 | - | - |
| BC-33 | 0 | 0 | 90 | 90 | - | + + + |
| BC-34 | 0 | 0 | 80 | 90 | - | + + |
| BC-35 | 0 | 0 | 90 | 90 | - | + + + + |
| BC-L | 0 | 0 | 0 | 0 | - | - |
| BC-36 | 0 | 0 | 75 | 90 | - | + + + |
| BC-37 | 90 | 85 | 90 | 90 | + + + | + + + + |
| BC-38 | 0 | 0 | 80 | 60 | - | + + |
| BC-39 | 0 | 0 | 75 | 70 | - | + + |

Notes: T = only slight color reaction, not considered positive.

The percent germination correlated well between benomyl (BRA) media and potato dextrose agar and benomyl (BRA) media. The color reactions also corresponded to the percent germination with more germination yielding greater color reactions. All isolates yielding a positive color reaction on vinclozolin (VRA) or benomyl (BRA) media were also resistant on the corresponding potato dextrose agar vinclozolin (PDAV) or potato dextrose agar benomyl (PDAB) media. Occasionally a spore overdose apparently allows a slight spore germination and slight color reaction to occur from sensitive isolates (BC-30-32 on vinclozolin media). These, however, can readily be detected visually by the lack of any mycelial growth and only a faint color reaction, not sufficient to be rated positive.

Although resistant strains of fungi can be obtained through field sampling techniques, samples of resistant strains are available upon request by contacting Plant Pathology, 26 Davis Drive, Research Triangle Park, N.C. 27709.

**Claims**

1. A diagnostic media for detecting fungicide resistant fungal plant pathogens comprising a dye, a fungicide, a carbohydrate and water, wherein the dye is an acid-base indicator dye and the fungicide is selected from the group consisting of two-ring dicarboximides, benzimidazole carbamates, and biscarbamates.

2. The diagnostic media of claim 1 further including a broad spectrum antibiotic.

3. The diagnostic media of claim 1 further including agar.

4. The diagnostic media of claim 1 further including at least one selective fungal growth inhibitor.

EP 0 373 543 B1

5. The diagnostic media of claim 1 wherein said acid-base indicator dye is selected from bromcresol purple and bromothymol blue.

6. The diagnostic media of claim 1 wherein said fungicide is selected from two-ring dicarboximide and benzimidazole carbamates.

7. A method for detecting fungicide resistant fungal plant pathogens comprising innoculating with fungal spores a diagnostic media comprising a dye, a fungicide, a carbohydrate and water; incubating said innoculated media; and macroscopically observing the media to detect the presence of fungicide resistant fungal plant pathogens, wherein the dye is an acid-base indicator dye, the fungicide is selected from the group consisting of two-ring dicarboximides, benzimidazole carbamates, and biscarbamates and the detection of fungicide resistant fungal plant pathogens is made by observing a color change of the media.

8. The method according to claim 7 wherein said diagnostic media further includes a broad spectrum antibiotic.

9. The method according to claim 7 wherein said diagnostic media further includes agar.

10. The method according to claim 7 wherein said diagnostic media further includes at least one selective fungal growth inhibitor.

**Patentansprüche**

1. Diagnostisches Medium zum Nachweis von fungizidresistenten pilzlichen Pflanzenkrankheitserregern mit einem Farbstoff, einem Fungizid, einem Kohlenhydrat und Wasser, bei dem der Farbstoff ein Säure-Basen-Indikatorfarbstoff ist und das Fungizid der Gruppe der Zweierring-Dicarbonsäureimide, Benzimidazolcarbamate und Biscarbamate gehört.

2. Diagnostisches Medium nach Anspruch 1, das weiterhin ein Breitbandantibiotikum enthält.

3. Diagnostisches Medium nach Anspruch 1, das weiterhin Agar-Agar enthält.

4. Diagnostisches Medium nach Anspruch 1, das weiterhin mindestens einen selektiven Hemmstoff gegen Pilzwachstum enthält.

5. Diagnostisches Medium nach Anspruch 1, das als Säure-Basen-Indikatorfarbstoff Bromkresolpurpur oder Bromthymolblau enthält.

6. Diagnostisches Medium nach Anspruch 1, wobei das Fungizid zur Gruppe der Zweierring-Dicarbonsäureimide und Benzimidazolcarbamate gehört.

7. Verfahren zum Nachweis von fungizidresistenten pilzlichen Pflanzenkrankheitserregern, dadurch gekennzeichnet, daß man ein diagnostisches Medium mit einem Farbstoff, einem Fungizid, einem Kohlenhydrat und Wasser mit pilzlichen Sporen inokuliert, dieses inokulierte Medium bebrütet und das Medium makroskopisch auf fungizidresistente pilzliche Pflanzenkrankheitserreger auswertet, wobei der Farbstoff ein Säure-Basen-Indikatorfarbstoff ist, das Fungizid zur Gruppe der Zweierring-Dicarbonsäureimide, Benzimidazolcarbamate und Biscarbamate gehört und fungizidresistente pilzliche Pflanzenkrankheitserreger durch einen Farbumschlag des Mediums nachgewiesen werden.

8. Verfahren nach Anspruch 7, wobei das diagnostische Medium weiterhin ein Breitbandantibiotikum enthält.

9. Verfahren nach Anspruch 7, wobei das diagnostische Medium weiterhin Agar-Agar enthält.

10. Verfahren nach Anspruch 7, wobei das diagnostische Medium weiterhin mindestens einen selektiven Hemmstoff gegen Pilzwachstum enthält.

14

**Revendications**

1. Milieu de diagnostic pour détecter des agents phytopathogènes fongiques résistant à un fongicide, comprenant un colorant, un fongicide, un glucide et de l'eau, dans lequel le colorant est un colorant indicateur acide-base et le fongicide est choisi dans le groupe formé par les dicarboximides bicycliques, les benzimidazole-carbamates et les biscarbamates.

2. Milieu de diagnostic de la revendication 1, comprenant de plus un antibiotique à large spectre.

3. Milieu de diagnostic de la revendication 1, comprenant de plus de la gélose.

4. Milieu de diagnostic de la revendication 1, comprenant de plus au moins un inhibiteur sélectif de croissance fongique.

5. Milieu de diagnostic de la revendication 1, dans lequel ledit colorant indicateur acide-base est choisi parmi le pourpre de bromocrésol et le bleu de bromothymol.

6. Milieu de diagnostic de la revendication 1, dans lequel ledit fongicide est choisi parmi les dicarboximides bicycliques et les benzimidazole-carbamates.

7. Méthode pour détecter des agents phytopathogènes fongiques résistant à un fongicide, consistant à ensemencer avec des spores fongiques un milieu de diagnostic comprenant un colorant, un fongicide, un glucide et de l'eau ; faire incuber ledit milieu ensemencé ; et observer le milieu au niveau macroscopique pour détecter la présence d'agents phytopathogènes fongiques résistant au fongicide, dans laquelle le colorant est un colorant indicateur acide-base, le fongicide est choisi dans le groupe formé par les dicarboximides bicycliques, les benzimidazole-carbamates et les biscarbamates, et la détection des agents phytopathogènes fongiques résistant au fongicide est effectuée par observation d'un changement de couleur du milieu.

8. Méthode selon la revendication 7, dans laquelle ledit milieu de diagnostic comprend de plus un antibiotique à large spectre.

9. Méthode selon la revendication 7, dans laquelle ledit milieu de diagnostic comprend de plus de la gélose.

10. Méthode selon la revendication 7, dans laquelle ledit milieu de diagnostic comprend de plus au moins un inhibiteur sélectif de croissance fongique.